# EUROPEAN PATENT APPLICATION

(11) **EP 2 508 201 A1**
(43) Date of publication of application: **10.10.2012**
(21) Application number: 10834263.5
(22) Date of filing: 29.11.2010
(51) Int. Cl.: A61K 39/10

(54) **METHOD FOR THE IDENTIFICATION OF ANIMALS VACCINATED AGAINST BRUCELLA**

(30) Priority: 03.12.2009 ES 200931101
(71) Applicant: Consejo Superior De Investigaciones Científicas (CSIC), 28006 Madrid (ES); Universidad Publica de Navarra, 31006 Pamplona (ES); Universidad Nacionalde Costa Rica (UNA), Costa Rica 86-3000 (CR); Universidad De Costa Rica (UCR), Montes de Oca, San José 2060 (CR)
(72) Inventor: GRILLÓ DOLSET, María, E-31192 Aranguren (Navarra) (ES); AMORENA ZABALZA, Beatriz, E-31192 Aranguren (Navarra) (ES); DE ANDRÉS CARA, Damián, E-31192 Aranguren (Navarra) (ES); MORENO ROBLES, Edgardo, Costa Rica 86-3000 (CR); CHAVES OLARTE, Estebán, Montes de Oca San José 2060 (CR); GUZMÁN VERRI, Caterina, Costa Rica 86-3000 (CR); CHACÓN DIAZ, Carlos, San José 2060 (CR)
(74) Representative: Pons Ariño, Angel
(86) International application number: PCT/ES2010/070784
(87) International publication number: WO 2011/067446

(57) **Abstract**

The present invention relates to: the use of a strain of Brucella spp. expressing the green fluorescent protein (GFP) in the preparation of medicaments for the prevention of brucellosis in mammals, vaccines against brucellosis, and the method for identifying mammals to which said vaccines have been administered. Preferably, the method of the invention is an immunoassay, and, more preferably, it is an enzyme-linked immunosorbent assay (ELISA).

## Description

The present invention is within the field of Preventive Medicine and Public Health, and relates both to the development and use of genetically modified live vaccines of *Brucella spp.,* as well as the development and use of direct and indirect diagnostic techniques that allow identification of animals that have been vaccinated and those which are infected by virulent strains of *Brucella spp.*

### STATE OF THE ART

Brucellosis is a disease transmitted from animals to humans. In animals, *Brucella* infection causes abortions, infertility, decreased production and limitations of the trading of animals and animal products, which is a problem for Animal Health with economic impact. In addition, the bacteria are transmitted from infected animals to humans, thereby inflicting a debilitating and often disabling disease, against which there is no vaccine and whose treatment requires high doses of antibiotics for prolonged periods with frequent relapses.

Therefore, brucellosis is a significant public health problem. It has been shown that the prevalence of human brucellosis is directly related to the prevalence of animal brucellosis. Therefore, and in the absence of vaccines for use in humans, prevention of the disease necessarily requires the control of the infection in animals. In most socio-economic contexts, the only feasible way to control brucellosis is through programmes based on the vaccination of farm animals, either through mass vaccination programmes or through programmes for the vaccination, diagnosis and slaughter of infected animals (Blasco 1997. Preventive Veterinary Medicine 31: 275 - 283).

The reference vaccines against animal brucellosis are *B. abortus* S19 (smooth strain) for cattle and *B*. *melitensis* Rev1 (smooth strain) for sheep and goats (OIE Terrestrial Manual, 2009 - chapters 2.4.3. and 2.7.2. -). Both are live attenuated vaccines, adjuvant-free, with a low cost of production and acquisition, and highly effective against infection by field strains in ruminants (main source of infection for humans). However, the technical drawback is that they generate an immune response after vaccination, indistinguishable from that induced after virulent infection by field strains. To solve this problem, numerous scientific efforts have been made. One strategy has consisted in the development of rough strains (R) of *Brucella,* which, due to the absence of the O chain of lipopolysaccharide (LPS) - a known virulence factor of *Brucella* and the main antigen used in tests for serological diagnosis of infection- has led to attenuated strains usable as live vaccines, which do not significantly interfere in the serological diagnostic tests. In this context, in the 90's, the spontaneous mutant with an R phenotype known as *B*. *abortus* RB51 was developed (by subculturing) (Schurig et al., 1991. Veterinary Microbiology, 28: 171 - 188). Strain RB51 is currently being used in some countries against bovine brucellosis, with controversial results. Both RB51 and a collection of R mutants derived from *B*. *melitensis* genetically well characterized in the different lipopolysaccharide synthesis pathways (Gonzalez et al., 2008. PLoS One, 3 (7): e2760), reduce interference problems in the serological diagnosis of virulent infection, due to the absence of "O" antigen. However, it has been shown that R vaccines do not solve the problem, because the protection they confer against field infections is well below that of the reference vaccines *B*. *abortus* S19 and *B. melitensis* Rev1 (Moriyón et al., 2005. Veterinary Research, 35: 1 - 38, Barrio et al., 2009. Vaccine, 27: 1741 - 1749). Therefore, it is essential to have genetically modified derived vaccines *B. abortus* S19 and *B melitensis* Rev1 and associated diagnostic tests to distinguish, through direct and indirect diagnosis (serology), between animals that have been vaccinated and those suffering from the virulent infection.

There is a wide variety of fluorescent proteins GFP (Green Fluorescent Protein), which are generated in variable fluorescence quantity and intensity depending on the expression system used. GFP proteins have been widely used in Molecular and Cellular Biology for genetic marking and detection of microorganisms (including *Brucella,* Celli et al., 2003. Journal of Experimental Medicine, 198 (4): 545 - 556), cells, plasmids, recombinant proteins and other elements for strictly scientific purposes. Immunoblotting-type immunological tests have also been developed (Rajasekaran et al., 2008. Applied and Environmental Microbiology, 74 (22): 7051 - 7055) as well as the sandwich ELISA for the detection and quantification of various GFP proteins expressed in microorganisms, cells and tissues (Cell Biolabs, Inc.; Cell Signaling Technology, Inc.). There are also PCR-type molecular diagnostic assays to identify specific variants of the gene encoding the GFP protein in bacteria such as *E. coli* (Clontech Laboratories) or *Staphylococcus epidermidis* (Franke et al., 2007. Journal of Microbiological Methods 71: 123 - 132).

Walsh and colleagues (Walsh et al., 2000. Journal of General Virology, 81, 709 - 718) proposed the possible use of a GFP as a veterinary vaccine marker. However, they failed to adequately express it in the Rinderpest virus.

### DESCRIPTION OF THE INVENTION

The authors of the present invention describe how a derivative strain of *B. abortus* S19 (the prototype vaccine S19-GFPp) is capable of expressing GFP in a stable manner, without altering the microbiological or biological characteristics (attenuation and efficacy against infection in experimental animals) of the reference strain S19 and is also able to induce anti-GFP antibody response detectable by specific serological tests that can differentiate the hosts which have been vaccinated and those infected with virulent strains of *Brucella* spp. They also describe an indirect ELISA method of serodiagnosis capable of specifically identifying animals that have been immunized with the new vaccine that expresses the GFP protein.

Therefore, a first aspect of the invention relates to the use of a *Brucella* spp. strain stably expressing the Green Fluorescent Protein (GFP) and being capable of eliciting an immune response in the host comparable to that induced by the reference vaccine strains and which also generates anti-GFP antibodies detectable by specific serological methods in the production of a medicament; or, alternatively, a *Brucella* sp. strain stably expressing the Green Fluorescent Protein (GFP) and being capable of eliciting an immune response in the host comparable to that induced by the reference vaccine strains and which also generates anti-GFP antibodies detectable by specific serological methods for its use as a medicament. In a preferred embodiment of this aspect of the invention, the medicament is a vaccine.

Another aspect of the invention relates to the use of a *Brucella* spp. strain stably expressing the Green Fluorescent Protein (GFP) and which is capable of eliciting an immune response in the host comparable to that induced by the reference vaccine strains and which also generates anti-GFP antibodies detectable by specific serological methods, in the production of a medicament for the prevention or treatment of brucellosis in a mammal; or, alternatively, a *Brucella* spp. strain stably expressing the Green Fluorescent Protein (GFP) and which is capable of eliciting an immune response in the host comparable to that induced by the reference vaccine strains and which also generates anti-GFP antibodies detectable by specific serological methods for use in the prevention or treatment of brucellosis in a mammal.

Herein, *Brucella* spp. refers to any cellular organism that can be defined as taxonomically belonging to the domain: *Bacteria,* phylum: *Proteobacteria,* class: *Alphaproteobacteria,* order: *Rhizobiales,* family: *Brucellaceae* and genus: *Brucella.* The term "mammal" means any organism of the domain: *Eukaryota,* kingdom: *Metazoa,* phylum: *Chordata,* subphylum: *Craniata,* superclass: *Gnathostomata* and class: *Mammalia.* Ruminant means any mammal belonging to the superorder: *Laurasiatheria,* suborder: *Ruminantia.* And bovine, ovine and caprine refer to any mammal that can be classified as belonging to the family: Bovidae.

The green fluorescent protein GFP produced by the jellyfish *Aequorea* sp. *(A victoria, A. Aequorea, A. forskalea)* is a protein that emits bioluminescence in the green area of the visible spectrum. In the context of the present invention, GFP is also defined by a nucleotide or polynucleotide sequence, which is the sequence encoding the GFP protein, and which would comprise several variants originating from:
a) nucleic acid molecules encoding a polypeptide comprising the amino acid sequence of the SEQ ID NO: 1,
b) nucleic acid molecules whose complementary strand hybridizes to the polynucleotide sequence of a),
c) nucleic acid molecules whose sequence differs from a) and / or b) due to the degeneracy of the genetic code,
d) nucleic acid molecules encoding a polypeptide comprising the amino acid sequence identical in at least 80 %, 90 %, 95 %, 98 % or 99 % to SEQ ID NO: 1, wherein the polypeptide encoded by said nucleic acids has the activity and the structural characteristics of the GFP protein.

In another preferred embodiment of the invention, the *Brucella* spp. strain belongs to the species *B. abortus.* In an even more preferred embodiment of this aspect of the invention, the *Brucella* strain is a strain derived from the reference strain *B. abortus* S19 (OIE Terrestrial Manual, 2009 - Chapter 2.4.3. - ). In another preferred embodiment, the *Brucella* strain belongs to the species *B. melitensis.* In an even more preferred embodiment of this aspect of the invention, the *Brucella* strain is a strain derived from the reference strain B. *melitensis* Rev 1 (OIE Terrestrial Manual, 2009 - Chapter 2.7.2. -).

*"Brucella abortus S19"* or *"Brucella abortus bv. 1 str. S19"* is a spontaneously attenuated strain discovered by Dr. John Buck in 1923, which has been used worldwide since early 1930's, as an effective vaccine to prevent brucellosis in animals and is internationally considered the reference vaccine for the control of bovine brucellosis (OIE Terrestrial Manual, 2009 - chapter 2.4.3. - ). The original seed lot is available at the U.S. Department of Agriculture (USDA, National Veterinary Services Laboratories, NVSL-, 1800 Dayton Road, Ames, Iowa 50010, United States of America) and at the Reference Laboratory for brucellosis of the OIE of the Veterinary Laboratories Agency (VLA, Weybridge, New Haw, Addlestone, Surrey KT15 3NB, United Kingdom). The strain is also known as NCTC 8038 (http: / / www.broadinstitute.org / annotation / genome / brucella_group / GenomeDescriptions.html).

*"Brucella melitensis Rev 1", "Brucella melitensis bv. 1 str. Rev. 1*" is a strain known as NCCB V4a (http: / / www.broadinstitute.org / annotation / genome / brucella_group / GenomeDescriptions.html) spontaneously attenuated and obtained from the virulent strain *B. melitensis* 6056, through successive spontaneous mutations associated with streptomycin dependence and the subsequent reversal of that dependency (Herzberg and Elberg 1953. Journal of Bacteriology 66: 585 - 599; Herzberg and Elberg 1953. Journal of Bacteriology 66: 600 - 605.). Rev 1 strain has been used worldwide since the 50's as the only effective vaccine to prevent brucellosis in small ruminants, and is internationally considered the reference vaccine to control ovine and caprine brucellosis (OIE Terrestrial Manual, 2009 - chapter 2.7.2. -). The original seed lots of Rev1 are available at the Brucellosis Reference Laboratory of the OIE of AFSSA (94706 Maisons-Alfort, France) or at the European Pharmacopoeia (BP 907, 67029 Strasbourg Cedex 1, France).

In a preferred embodiment of the invention, the mammal is a ruminant. In another preferred embodiment of the invention, the mammal is bovine, and even more preferably belongs to the subfamily *Bovinae* or *Caprinae.*

Another aspect of the invention relates to a composition, hereinafter "composition of the invention", comprising a *Brucella* spp. strain expressing the GFP protein, and preferably a pharmaceutically acceptable carrier. In a preferred embodiment, the *Brucella* strain belongs to the species *B*. *abortus.* In an even more preferred embodiment of this aspect of the invention, the *Brucella* strain is a strain derived from *B*. *abortus* S19. In another preferred embodiment, *Brucella* strain belongs to the species *B. melitensis.* In a more preferred embodiment of this aspect of the invention, *Brucella* strain is a strain derived from *B. melitensis* Rev 1. In another preferred embodiment, the composition is a vaccine. In another more preferred embodiment, the composition of the invention further comprises an adjuvant. In another more preferred embodiment, the composition of the invention comprising a *Brucella* spp. strain expressing the GFP protein, further comprises another active ingredient.

The composition of the invention may be formulated for administration to an animal, and more preferably a mammal, including ruminants, in a variety of ways known in the prior art for use as an immunogen. These immunogens can also be used as vaccines in animals, and more particularly in mammals, or to produce a response in antibody production therein. For the formulation of such compositions, an immunologically effective amount of the strain of *Brucella* spp. is mixed with a physiologically acceptable carrier suitable for administration to mammals including humans. Thus, the composition of the invention may be found, but is not limited thereto, in a sterile aqueous solution or in biological fluids such as serum. The aqueous solutions may be buffered or unbuffered and have additional active or inactive components. Additional components include salts for modulating the ionic strength, preservatives including but not limited to, antimicrobial agents, antioxidants, chelators and the like, and nutrients including, but not limited to, glucose, dextrose, vitamins and minerals. Alternatively, the active ingredient can be prepared for administration in solid form. The active ingredient may be combined with various inert carriers or excipients, including but not limited to, binders such as microcrystalline cellulose, gum tragacanth or gelatin; excipients such as starch or lactose; dispersing agents such as alginic acid or corn starch; lubricants such as magnesium stearate, glidants such as colloidal silicon dioxide; sweetening agents such as sucrose or saccharin; or flavouring agents such as peppermint or methyl salicylate.

The composition of the invention can be administered to an animal, including a mammal, preferably a ruminant, and even more preferably belonging to subfamilies *Bovinae* or *Caprinae,* in a variety of forms, including but not limited to, intraperitoneal, intravenous, intramuscular, subcutaneous, conjunctival, intrathecal, intraventricular, oral, enteral, parenteral, intranasal or dermal administration.

The dosage to obtain a therapeutically effective amount depends on a variety of factors (e.g., age, weight, sex, physiological state -such as pregnant or lactating states- tolerance condition of the immune system) of the animal, preferably a mammal. As used herein, the term "therapeutically effective amount" refers to the amount of *Brucella* spp. stably expressing the GFP protein, producing the desired effect (the generation of immunity and anti-GFP antibodies). The "adjuvants" and "pharmaceutically acceptable carriers" which can be used in such compositions are known to those skilled in the art.

Herein, the term "medicament" means any substance used for the prevention, diagnosis, mitigation, treatment or cure of diseases in humans and animals. In the context of the present invention it relates to the composition comprising *Brucella* spp. strains stably expressing the GFP protein, and which are capable of generating immunity against brucellosis and anti-GFP antibodies, or to the composition comprising *Brucella* spp. stably expressing the GFP protein and which is able to generate immunity against Brucellosis and anti-GFP antibodies and a pharmaceutically acceptable carrier and / or additionally, an adjuvant. The term medicament therefore includes vaccines.

In the context of the present invention the term "vaccine" refers to an antigenic composition or preparation used to establish the response of the immune system to a disease. It refers to antigen preparations that once inside the body cause the response of the immune system by antibody production, and immunological memory is generated producing permanent or transient immunity.

In this specification, the term "adjuvant" refers to an agent which, while not possessing in itself an antigenic effect, can stimulate the immune system by increasing its response to the vaccine. Although without being limited to the following, the aluminium salts "aluminium phosphate" and "aluminium hydroxide" are the two most commonly used adjuvants in vaccines. Other substances, such as squalene, can also be used as adjuvants.

As used herein, the term "active principle", "active substance", "pharmaceutically active substance", "active ingredient" or "pharmaceutically active ingredient" means any component which provides a potential pharmacological activity or another different effect in the diagnosis, cure, mitigation, treatment, or prevention of a disease, or which affects the structure or function of the human body or other animals. The term includes those components that promote a chemical change in the preparation of the drug and are present therein in a foreseen modified form that provides the specific activity or effect.

Another aspect of the invention relates to a method for the identification of mammals vaccinated with the strain or the composition of the invention, hereinafter "method of the invention", comprising:
a) obtaining an isolated biological sample from the mammal,
b) detecting the presence of the *gfp* gene encoding the GFP protein, or their expression products, in an isolated mammalian biological sample.

In a preferred embodiment of this aspect of the invention, the mammal is a ruminant. In another preferred embodiment of the invention, the mammal is bovine, ovine or caprine, and even more preferably belonging to the subfamilies *Bovinae* and *Caprinae.*

An "isolated biological sample" includes, without being limited to, cells, tissues and / or biological fluids of a mammal, obtained by any method known to one skilled in the art. In a preferred embodiment, the isolated biological sample is a biological fluid, such as, but not limited to, milk, semen, seminal fluid, vaginal discharge, conjunctival discharges, blood, plasma or blood serum. More preferably, the biological fluid is blood serum. In another preferred embodiment, the isolated biological sample is cells found in blood, milk, semen, vaginal discharges or conjunctival secretions of the mammal. In another preferred embodiment, it includes cells or tissues.

Numerous methods for detecting the presence of the *gfp* gene or its expression products in the isolated biological sample are known in the state of the art. Herein "expression products of the *gfp* gene" include, but are not limited to, both the GFP protein, such as anti-GFP antibodies generated by the immune system of mammals against said protein or antigen. Thus, in another preferred embodiment of this aspect of the invention, detection of the presence of the *gfp* gene or its expression products in the isolated mammalian biological sample is performed by detecting the antibodies against the GFP protein (anti-GFP antibodies). Detection of anti-GFP antibodies may be performed by any method known in the prior art for example, but not limited to, by immunoassay or immunohistochemistry. In a more preferred embodiment, the immunoassay is an enzyme-linked immunosorbent assay or ELISA.

An antigen or immunogen is a substance capable of producing an adaptive immune response by activating lymphocytes. Antigens are usually proteins or polysaccharides. Lipids and nucleic acids are antigenic only when combined with proteins and polysaccharides. The term "antibody" as used herein refers to immunoglobulin molecules and immunologically active portions of immunoglobulin molecules, i.e., molecules that contain an antigen binding site which specifically binds (immunoreacts) with the GFP protein (antigen). There are five major isotypes or classes of immunoglobulins: IgM, IgD, IgG, IgA and IgE.

The term "anti-GFP antibody" refers to an antibody capable of reacting with the GFP protein, with a GFP protein variant or a fragment thereof, provided that said variant or fragment is functionally equivalent. Preferably, the term anti-GFP antibody refers to an immunoglobulin G (IgG).

The term "immunoassay", as used in the present description, refers to any analytical technique that is based on the reaction of the conjugation of an antibody with an antigen. Examples of immunoassays known in the prior art are, but not limited to: immunoblotting, enzyme-linked immunosorbent assay (ELISA), line immunoassay (LIA), radioimmunoassay (RIA), immunofluorescence, x-map or protein or lipopolysaccharide chips (LPS). As said, in a preferred embodiment of this aspect of the invention, the immunoassay is an enzyme-linked immunosorbent assay or ELISA (Enzyme-Linked ImmunoSorbent Assay). ELISA is based on the assumption that an immunoreagent (antigen or antibody) can be immobilized on a solid support, then putting this system in contact with a fluid phase containing the complementary reagent that can bind to a marker compound. There are different types of ELISA: direct ELISA, indirect ELISA and sandwich ELISA.

The authors of the present invention describe a method for serological diagnosis capable of identifying animals that have been vaccinated with the new vaccine prototype S19-GFPp and differentiate them from those infected with virulent strains of *Brucella* spp.

In a more preferred embodiment, the ELISA is an indirect ELISA, and even more preferably it comprises the following steps:
(a) coating a solid support with at least the GFP protein, a variant of said protein, or a fragment thereof;
(b) incubating the impregnated support from the preceding step with a biological sample obtained from the mammal under conditions allowing the formation of an immunocomplex of the antibodies at least against the GFP antigen, with variants or fragments thereof; and
(c) incubating with a secondary antibody, which recognizes the antibodies against the GFP antigen, conjugated or linked to a marker compound.

The term "marker compound" as used herein, refers to a compound capable of giving rise to a chromogenic, fluorogenic, radioactive and / or chemiluminescent signal allowing detection of the anti-GFP antibody. The marker compound is selected from the list comprising radioisotopes, enzymes, fluorophores or any molecule capable of being combined with another molecule or detected and / or quantified directly. The marker compound can bind directly or indirectly to the antibody by means of another compound. Examples of marker compounds that directly bind are, but not limited to, enzymes such as alkaline phosphatase or peroxidase, radioisotopes such as ₃₂P or ₃₅S, fluorochromes such as fluorescein, rhodamine or derivatives thereof, or metal particles, for direct detection by colorimetry, autoradiography, fluorimetry, or metallography respectively.

In another preferred embodiment, detection of the presence of the *gfp* gene or its expression products in the isolated mammalian biological sample is performed by ultraviolet illumination of said sample. In another more preferred embodiment, detection of the presence of the *gfp* gene or its expression products in the isolated mammalian biological sample is performed by fluorescence microscopy.

In another preferred embodiment, detection of the presence of the *gfp* gene or its expression products in the isolated mammalian biological sample is performed by polymerase chain reaction (PCR).

Another aspect of the invention relates to a "kit" for identifying mammals vaccinated with the strain or the composition of the invention, comprising suitable means for carrying out the method of the invention. Said kit may contain all those reagents necessary to analyze the presence of the *gfp* gene or its expression products in the isolated mammalian biological sample using any of the methods described hereinabove, for example, but not limited to, specific antibodies of the GFP protein, or secondary antibodies or positive and / or negative controls. The kit may also include, but is not limited to, buffers, protein extraction solutions, agents to prevent contamination, protein degradation inhibitors, etc. In the case of detection by techniques which involve the polymerase chain reaction (PCR) it may contain, but is not limited to, primers, probes and all reagents necessary to determine the presence of the *gfp* gene or its expression products. The kit may also include, but is not limited to, the use of buffers, polymerase, cofactors to obtain an optimal activity thereof, agents to prevent contamination, etc... On the other hand, the kit may include all support means and containers required for its putting into practice and optimization. Preferably, the kit further comprises instructions for carrying out the methods of the invention.

The terms "polynucleotide" and "nucleic acid" are used herein interchangeably, referring to polymeric forms of nucleotides of any length, both ribonucleotides (RNA) and deoxyribonucleotides (DNA).

The terms "amino acid sequence", "peptide", "oligopeptide", "polypeptide" and "protein" are used herein interchangeably, and refer to a polymeric form of amino acids of any length chemically or biochemically modified.

Throughout the description and claims the word "comprise" and its variations are not intended to exclude other technical characteristics, additives, components or steps. For those skilled in the art, other objects, advantages and characteristics of the invention will become apparent partly from the specification and partly from the practice of the invention. The following examples and drawings are provided by way of illustration and are not intended to limit the scope of the present invention.

### DESCRIPTION OF THE FIGURES

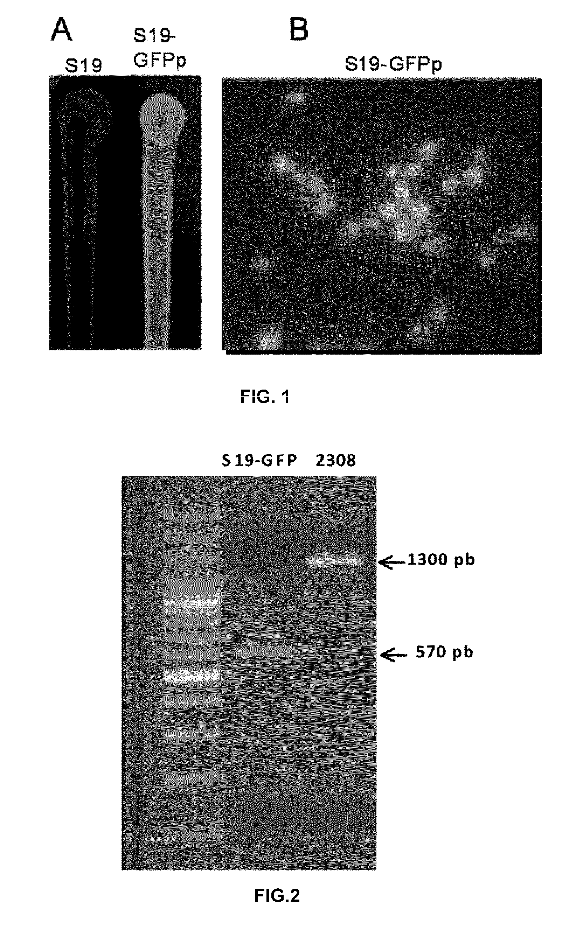

**Figure 1****. Growth of the new vaccine-GFPp S19** and of the parental strain S19 in trypticase soy agar plates illuminated with ultraviolet light in a "Gel Doc" device by Bio Rad and visualized with the appropriate filter (520DF30 nm, Bio Rad) (A) or in a fluorescence microscope (B).
**Figure 2****. PCR amplification of the *ery* region in the new vaccine S19-GFPp** (low molecular weight) compared with that of the virulent strain *B*. *abortus* 2308.

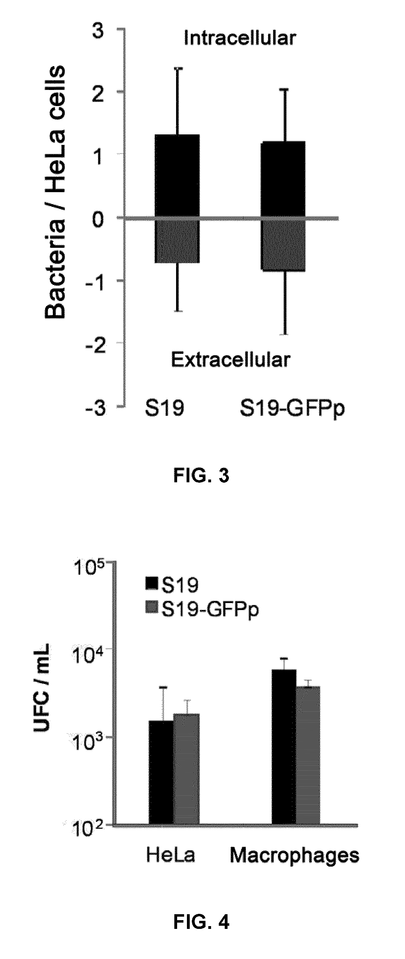

**Figure 3****. Ratio of intracellular and extracellular bacteria in HeLa cells infected with the new vaccine S19-GFPp** or with the parental strain S19, one hour after infection. HeLa cells were infected with 200 CFU / cell and 1 hour later, were fixed and labelled by double immunofluorescence with rhodamine and FITC, following the protocols previously described (Chaves-Olarte et al., 2002. Cellular Microbiology 4 (10): 663 - 675).
**Figure 4****. Replication of the new vaccine S19-GFPp and of the parental strain S19 in HeLa cells and RAW 264.7 macrophages at 48 hours** post-infection. Cells were infected with 200 CFU / cell and the number of CFU / mL was determined, following the protocols previously described (Chaves-Olarte et al., 2002. Cellular Microbiology 4 (10): 663 - 675; Celli et al., 2003. Journal of Experimental Medicine, 198 (4): 545 - 556).

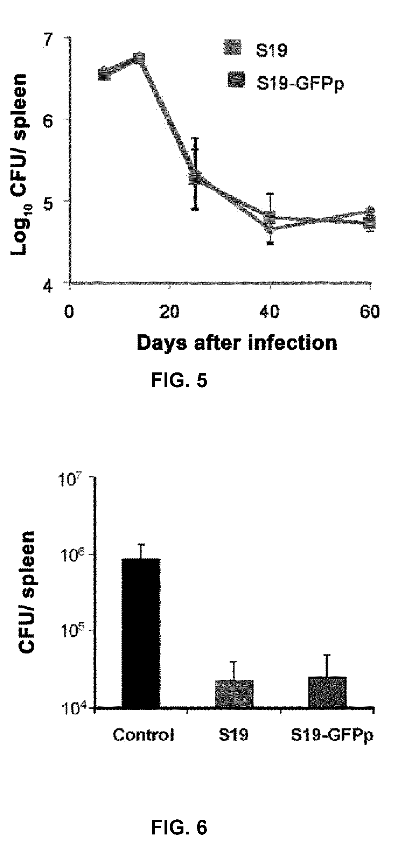

**Figure 5****. Splenic kinetics of the new vaccine S19-GFPp and of a parental strain S19 in the murine model.** Groups of 30 mice were inoculated intraperitoneally with 1x10⁵ CFU of the new vaccine S19-GFPp or the parental strain S19. At 7, 14, 25, 40 and 60 days after infection, six mice from each group were sacrificed to determine the number of CFU / spleen of each vaccine strain and construct the corresponding curves of splenic multiplication, following the protocols previously described (Sangari et al., 1998. Vaccine, 16 (17): 1640 - 1645).
**Figure 6****. Protection assay in BALB / c mice immunized with the new vaccine S19-GFPp**. Groups of 6 mice were immunized subcutaneously with 1x10⁵ CFU of the new vaccine S19-GFPp or the parental strain S19. As a control, a group of mice was inoculated with sterile PBS. After 60 days, all mice were experimentally infected intraperitoneally with 5x10⁴ CFU of the reference virulent strain *B*. *abortus* 2308 and 2 weeks later, were sacrificed to count the number of CFU of 2308 per spleen. Animals vaccinated with S19-GFPp showed similar levels of protection to those shown by the reference vaccine S19.

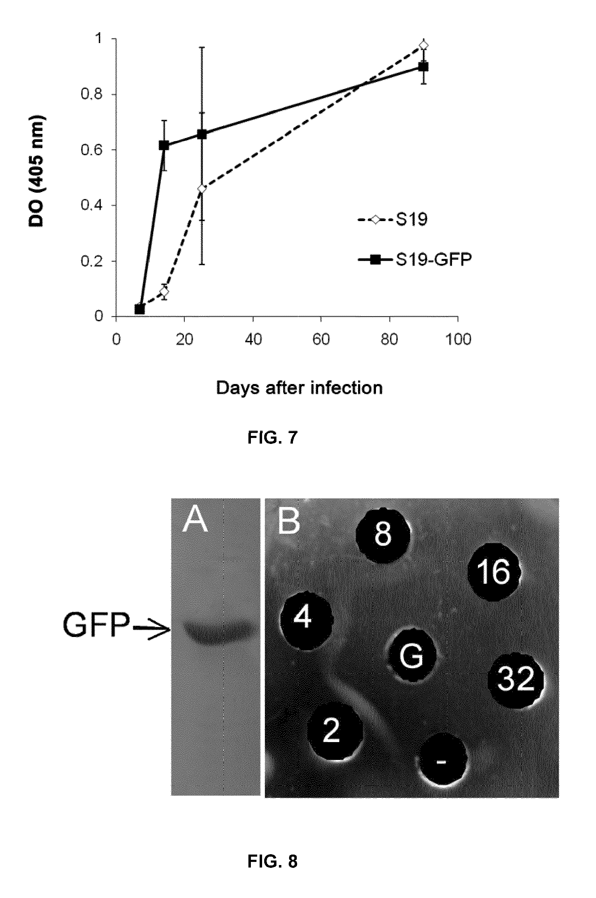

**Figure 7****. Antibody response against *Brucella* LPS in mice immunized with the new vaccine S19-GFPp and the parental strain S19.** Serum samples were obtained in non-immunized mice (controls) and in mice immunized intraperitoneally with 1x10⁵ CFU of the new vaccine S19-GFPp or the parental strain S19, at 7, 14, 25 and 60 days after the immunization. These sera were diluted 1 / 200 in PBS and their Optical Density (OD) was measured at 405 nm in an indirect ELISA against LPS customarily used for diagnosis of animal brucellosis. Each point on the graph represents the average based on 5 sera from different mice. All animals developed antibodies against *Brucella* LPS.
**Figure 8****. Determination of the degree of purity and immunogenicity in mice of the recombinant protein GST-GFP obtained. *A*) The recombinant** protein GST-GFP purified by affinity, showed a single band in SDS-PAGE. B) The GST-GFP protein used as immunogen in mice induced anti-GFP antibodies. The immunodiffusion reaction was performed with serial dilutions (wells 2 to 32) of monospecific serum against 10 µg / 30 µL of GFP (well G) and, as a control, PBS alone was used (well -). The precipitation bands show that the anti-GFP immune serum obtained in mice showed a titre of 1 / 16.

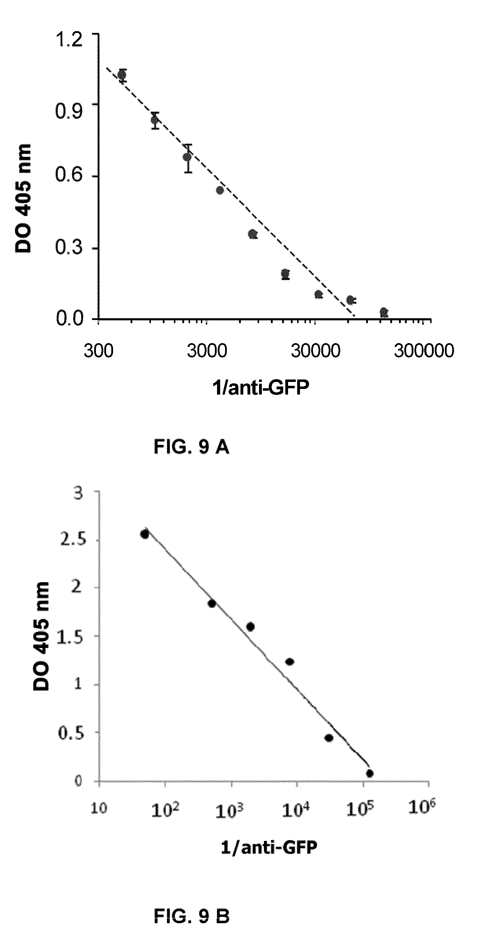

**Figure 9****. Standard curves in the ELISA-GFP developed, using sera from mice (a) or sheep (b) previously immunized with the recombinant protein GST-GFP).**

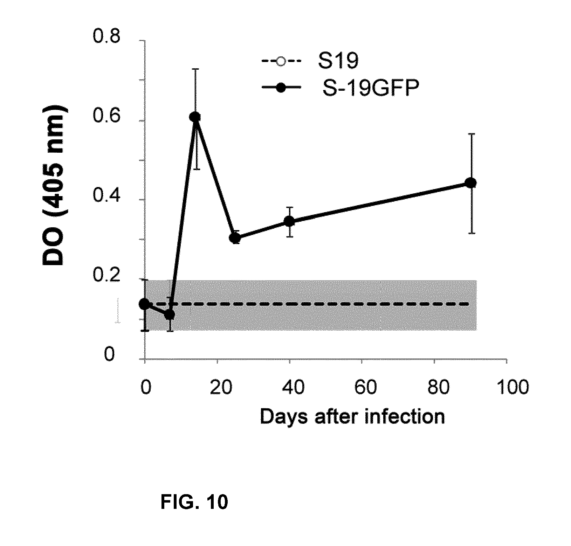

**Figure 10****. Intensity of the antibody response against GFP in sera from mice inoculated with the new S19-GFPp vaccine or the reference vaccine S19.** The intensity of the reaction of each individual serum was calculated according to the percentage of positivity of the sample as regards to the control (OD of positive serum / OD of the serum sample X 100). Each point represents the average based on 10 sera from different mice.

### EXAMPLES

The invention will be illustrated hereunder by means of assays performed by the inventors.

### 1 - Development of a prototype of fluorescent vaccine derivative of B. abortus S19 (S19-GFPp).

The reference vaccine *B*. *abortus* S19 (obtained from the Brucellosis Reference Laboratory of the OIE of AFSSA, France) was genetically modified by electroporation with a plasmid encoding the GFP. The plasmid used was pBBR-2-gfp, derived from the plasmid pBBRIMCS-2, which contains a kanamycin resistance insert (Kovach et al., 1995. Gene. Vol 166: 175 - 176) and an insert with the gene encoding the GFP under the control of the lac promoter. It is known that this plasmid constitutively produces GFP, without being integrated into the *Brucella* chromosome (Celli et al., 2003. Journal of Experimental Medicine, 198 (4): 545 - 556).

### 2. - Genetic and microbiological characterization of S19-GFPp.

**2.1**. It was found that the bacteria S19-GFPp emit fluorescence detectable by direct illumination of the cultures with ultraviolet light and subsequent visualization thereof with a suitable filter (Fig. 1A) or by observation of infected tissues or exudates under a fluorescence microscope (Fig. 1 B). Therefore, the plasmid pBBR-2-gfp allows expression of GFP in *Brucella* in suitable proportions so that the new vaccine S19-GFPp can be visually identified after isolation.
**2.2.** It was found that the expression of GFP protein in *Brucella* does not alter the colonial size and phase of S19 in bacterial cultures, or its classical bacteriological characteristics. To this end, the colony size was determined by measuring the diameter of the colonies obtained on agar plates after 3 days of incubation. In parallel, the colonial phase was determined by observation of bacterial growth in an oblique illumination lens and staining using the flooding technique with a solution of crystal violet-oxalate (Alton et al. 1988. Techniques for the brucellosis laboratories. In. INRA (Ed.), Paris, France, 1988, 190 pp).
   In addition, the new strain obtained was microbiologically analyzed using standard techniques for the identification and typing of *Brucella* spp. Briefly, the genus level identification was performed by testing for catalase, oxidase, urease and acriflavine agglutination. For species-level identification, the test on sensitivity to bacteriophages Tb, Wb, Iz and R / C was used. Finally, for biovariety-level typing agglutination tests were used with monospecific sera anti-A and anti-M and growth tests on agar plates and with agar supplemented with 10 % sterile bovine serum (agar-S; Seromed, Biochrom) containing standard concentrations of dyes (20 µg / mL of thionine, 20 µg / mL of fuchsin and 100 µg / mL of safranine; Panreac), antibiotics (5 UI / mL of penicillin; Sigma) and erythritol (1 mg / mL; Merck). The plates containing agar and agar-S (control plates), with or without the different concentrations of all these products, were incubated for 2 - 4 days at 37 °C in an aerobic atmosphere, and in parallel, in an atmosphere with 10 % CO₂ (Alton et al. 1988. Techniques for the brucellosis laboratories. In. INRA (Ed.), Paris, France, 1988, 190 pp).
**2.3.** It was found that both the incorporation and activity of the plasmid carrying the *gfp* gene in strain S19-GFPp is stable after its activity *in vitro* and *in vivo.* In this regard, serial subculturing of the strain S19-GFPp on agar plates was performed and after several serial passages, bacterial counts were performed in agar plates and agar supplemented with kanamycin (antibiotic used as a plasmid marker). As a result, it was observed that the number of CFU in both culture media was similar and that all of S19-GFPp CFU maintained the fluorescence, indicating that incorporation and activity of the GFP plasmid in B. *abortus* S19 is stable after twelve subcultures *in vitro.* The same test was carried out after isolating the bacteria from four successive passages in cell cultures and three successive passages in tissue of mice previously inoculated with S19-GFPp (see biological characterization studies shown below), indicating that the incorporation and activity of the GFP plasmid in *B. abortus* S19 is stable even after the bacterial activity *in vivo,* in cell cultures and experimental animals (mice). The plasmids encoding the GFP protein remained stable in *B. abortus* S19, after freezing in 50 % glycerol at -80 °C or -20 °C, showing that all recovered colonies emitted fluorescence after thawing.
**2.4.** It was found that the strain S19-GFPp maintains the classic genotype of *B*. *abortus* S19, retaining the characteristic deletion of 702 bp (base pairs) in the gene *ery* (low molecular weight band, Fig 2) and makes it possible to differentiate the vaccine strain S19 from the virulent strains of *B. abortus* (higher molecular weight band, Fig 2).

### 3. - Biological characterization of S19-GFPp in cell and animal models (mice) widely used in experimental brucellosis.

**3.1.** In cellular models it was found that:
   **3.1.1** - the new vaccine S19-GFPp has a capacity of adhesion and internalization in human epithelial HeLa cells which is virtually identical to that of the reference vaccine (Fig. 3).
   **3.1.2 -** the replication capacity of the new vaccine S19-GFPp in HeLa cells and in macrophages is virtually identical to the parental strain S19 (Fig. 4).
**3.2**. In animal models it was found that:
   **3.2.1** - the new vaccine S19-GFPp possesses an attenuation in mice which is virtually identical to that of the classical vaccine S19 (Fig. 5), indicating that the *gfp* gene expression used does not alter the splenic kinetics (multiplication and persistence capacity) of the classical parental vaccine.
   **3.2.2** - the new vaccine S19-GFPp confers protection which is virtually identical to that conferred by the classic parental vaccine in the mouse model (Fig. 6), indicating that GFP expression does not diminish the effectiveness of the classical parental vaccine S19.
   **3.2.3** - the antibody response against the LPS antigen of *B. abortus* induced by the new vaccine S19-GFPp, measured by conventional indirect ELISA against LPS (Marin et al., 1999. Clinical & Diagnostic Laboratory Immunology 6 (2): 269 - 272), is similar to that generated by the classical vaccine S19 (Fig. 7). This shows that the new vaccine S19-GFPp retains its immunogenic properties intact.
   **3.2.4** - GFP expressed in the *Brucella* S19-GFPp vaccine strain is highly immunogenic in animals. All mice immunized with S19-GFPp develop specific antibodies against GFP for at least 90 days after vaccination, with a positivity of approximately 40 % (statistically greater than that shown by the positive control) in the ELISA-GFP prototype described herein.

### 4. - Design and standardization of an indirect ELISA to detect antibodies generated by S19-GFPp in immunized mice (ELISA-GFP).

**4.1**. First, the recombinant GFP protein (GST-GFP) was expressed and purified according to the protocols described previously (Harlow and Lane 1988. Antibodies: a laboratory manual. 1 st. Ed. Cold Spring Harbor Laboratory, NY p. 179 - 179). Briefly, the fusion protein GST-GFP was expressed in the system *E. coli* XL1-Blue with the plasmid pGEX-GFP, it was purified by affinity chromatography and its purity was determined by acrylamide gel electrophoresis (Fig. 8A).
**4.2.** Subsequently, control sera against GFP in mice were obtained, following the conventional immunization protocols. To this end, an injection of 100 µg of GST-GFP protein with Freund's complete adjuvant was administered, followed by 2 consecutive immunizations, with a one-week interval, with GST-GFP protein with Freund's incomplete adjuvant. Immunizations were performed for several weeks until verifying that the sera of animals immunized with GST-GFP showed precipitation bands in gel immunodiffusion against the purified GFP protein (Fig. 8B). These control antibodies showed no cross reactions with any *Brucella* antigen (results not shown).
   In addition, control sera against GFP in sheep were obtained, following the conventional immunization protocols. To this end, an injection of 100 µg of GST-GFP protein with Freund's complete adjuvant, followed by 5 consecutive immunizations, at two-week intervals, with GST-GFP protein with Freund's incomplete adjuvant. Immunizations were performed for two months until verifying that the sera of animals immunized with GST-GFP showed precipitation bands in gel immunodiffusion against the purified GFP protein (results not shown). These control antibodies showed no cross reactions with any *Brucella* antigen (results not shown).
**4.3.** For the standardization of indirect ELISA capable of specifically detecting antibodies against the GFP protein (ELISA-GFP) in sheep sera and in serum of mice vaccinated with the new vaccine S19-GFPp, the procedure was as follows:
   **4.3.1.** Coating the plates of 96 well plates Immunolon II (Nunc Co.) with 1 µg of GST-GFP per well, contained in a volume of 100 µL / well of a buffer solution of 0.01 % of Tween 20 in PBS (PBST, Sigma .) The plates were covered with adhesive plastic (Sigma) and incubated at 37 °C for 2 hours while stirring, followed by another incubation at 4 °C for 15 hours without stirring. Finally, 30 % glycerol was added to each well, the plates were covered with adhesive plastic and frozen at -20 °C until use.
   **4.3.2.** To establish the calibration curve with mice sera, the plates described in section 4.3.1. were allowed to thaw at room temperature and washed 4 times with a solution containing 0.01 % of Tween and 0.1 % of delipidated bovine albumin in PBS (PBST-BSA). Then, 100 µL per well of dilutions between 1 / 300 and 1 / 60000 of anti-GFP immune serum from mice described in section 4.2. were added in triplicate. As negative controls, conventional sera from *Brucella*-free mice were used and PBS as blank. The plates were incubated for 1 hour at 25 °C while stirring, were washed 4 times with PBST-BSA; a conjugate (obtained from rabbits) coupled to peroxidase (HRP) against the IgG (H+L) of mice was added; they were incubated (1 hour at 25 °C, while stirring) and washed (4 times) as described previously a second time, and, finally, ABTS peroxidase substrate (Sigma) was added. The reading of the OD generated in each well was performed at 405 nm wavelength in an ELISA reader and the standard curve was established as shown in Fig 9.a.
   **4.3.3**. To establish the calibration curve with sheep sera, the plates described in section 4.3.1. were allowed to thaw at room temperature and washed 4 times with a solution containing 0.01 % of Tween and 0.1 % of delipidated bovine albumin in PBS (PBST-BSA). Then, 100 µL per well of dilutions between 1 / 59 and 1 / 128000 of the anti-GFP immune serum from sheep described in point 4.X were added in triplicate. As negative controls, conventional sera from Brucella-free sheep were used, and PBS as blank. The plates were incubated for 1 hour at 37 °C while stirring, were washed 4 times with PBST; Protein G-peroxidase (Sigma) was added in dilution 1: 2000 in PBS pH 7.2; they were incubated (1 hour, at 25 °C while stirring) and washed (4 times) as described above a second time; and finally ABTS peroxidase substrate (Sigma) was added The reading of the OD generated in each well was performed at 405 nm wavelength in an ELISA reader and the standard curve was established as shown in Fig 9.b.
   **4.3.4.** To determine the intensity of antibody response against GFP induced in mice inoculated with the new vaccine, "problem" sera from mice inoculated (1x10⁵ CFU / mouse, intraperitoneally) with the new vaccine S19-GFPp or one from the unmarked parental strain S19 were used, and the animals were bled periodically, between 3 and 14 weeks post-immunization (Fig. 10). As positive controls sera were used as described in section 4.2., and as negative controls, those described in section 4.3.2. All these sera were diluted in a proportion of 1 / 200 in PBST and the ELISA-GFP was carried out as follows: the plates described in section 4.3.1. were washed as described in section 4.3.2.; then 100 µl per well of the sera described above (diluted 1 / 200) were added or 100 µl per well of PBS, as blank of the reaction, and; finally, the plates were processed as described in 4.3.2. The percentage of positivity for each individual serum was calculated with respect to the optical density (OD₄₀₅ = 0.9) of the positive control serum (100 % positive) and of the blank (0 % positive). All animals inoculated with S19-GFPp showed an intense serological reaction against GFP (about 40 % positivity; Fig. 10) while animals inoculated with strain S19 behaved like those without *Brucella* (negative controls) not reacting against the GFP protein (Fig. 10).

In conclusion, the work shows that the GFP protein can be expressed in *Brucella* vaccine strains without altering the biological properties of the parental strain, while inducing in animals a serological response clearly distinguishable from that induced by other *Brucella* strains. Antibodies generated against the GFP protein can be identified by indirect ELISA against GFP developed in this patent. Furthermore, the incorporation of the *gfp* gene in *Brucella* provides both visual identification (by ultraviolet light or fluorescence microscopy) and molecular identification (by a PCR amplifying the gfp gene; PCR-GFP) of *Brucella* vaccine strains developed from both bacterial cultures and tissue samples, exudates or animal fluids.

## Claims

1. Use of a *Brucella* spp. strain expressing the Green Fluorescent Protein (GFP) in the manufacture of a medicament.

2. Use of a *Brucella* spp. strain according to claim 1, in the development manufacture of a medicament for the prevention of brucellosis in mammals.

3. Use of a *Brucella* spp. strain according to any of claims 1 - 2, wherein the *Brucella* strain belongs to the species *B*. *Abortus.*

4. Use of a *Brucella* spp. strain according to claim 3, wherein the *Brucella* strain is a strain derived from the reference strain *B*. *abortus* S19.

5. Use of a *Brucella* spp. strain according to any of claims 1 - 2, wherein the *Brucella* strain belongs to the species *B. melitensis.*

6. Use of a *Brucella* spp. strain according to claim 5, wherein the *Brucella* strain is a strain derived from the reference strain *B*. *melitensis* Rev 1.

7. Use of a *Brucella* spp. strain according to any of claims 2 - 6, wherein the mammal is a ruminant.
Use of a *Brucella* spp. strain according to claim 5, wherein the ruminant belongs to the subfamily Bovinae.

8. Composition comprising a *Brucella* spp. strain expressing the Green Fluorescent Protein (GFP) according to any of claims 1 - 9.

9. Composition according to the preceding claim further comprising a pharmaceutically acceptable carrier.

10. Composition according to any of claims 10 - 11, which is a vaccine.

11. Composition according to any of claims 10 - 12, further comprising an adjuvant.

13. Composition according to any of claims 10 - 12, further comprising another active ingredient.

14. Method for the identification of mammals treated with the composition according to any of claims 10 - 14, comprising:
a. obtaining an isolated biological sample from the mammal,
b. detecting the presence of the *gfp* gene or its expression products in the isolated biological sample from the mammal.

15. Method for the identification of mammals treated according to the preceding claim, wherein the isolated biological sample from the mammal is a biological fluid.

16. Method for the identification of mammals treated according to claim 15, wherein the isolated biological sample from the mammal is cells or tissues.

17. Method for the identification of mammals treated according to any of claims 15 - 17, wherein the detection of the expression products of the *gfp* gene in the isolated biological sample from the mammal is performed by the detection of anti-GFP antibodies.

18. Method for the identification of mammals treated according to claim 18, wherein the detection of the anti-GFP antibodies is performed by immunoassay.

19. Method for the identification of mammals treated with the strain or composition according to claim 19, wherein the immunoassay is an enzyme-linked immunosorbent assay (ELISA).

20. Method for the identification of mammals treated with the strain or composition according to claim 20, wherein the ELISA assay is an indirect ELISA.

21. Method for the identification of mammals treated with the strain or the composition according to any of claims 15 - 17, wherein the detection of the *gfp* gene or its expression products is performed by ultraviolet light.

22. Method for the identification of mammals treated with the strain or the composition according to any of claims 15 - 17 and 22, wherein the detection of the *gfp* gene or its expression products is performed by fluorescence microscopy.

23. Method for the identification of mammals treated with the strain or the composition according to any of claims 15 - 17, wherein the detection of the *gfp* gene or its expression products, is carried out by means of the polymerase chain reaction.

24. Kit for the identification of mammals treated with the strain or the composition according to any of claims 1 - 14, comprising suitable means for carrying out a method of identification according to any of claims 15 - 24.

26. Identification kit according to the preceding claim, comprising suitable means for detecting the anti-GFP antibodies.

27. Identification kit according to any of claims 25 - 26, comprising the primers with sequences SEQ ID NO: 2 and SEQ ID NO: 3.


SEQUENCE LISTING

<110>  Consejo Superior de Investigaciones Científicas (CSIC)
       Universidad de Costa Rica
       Universidad Nacional de Costa Rica
       Universidad Pública de Navarra

<120>  Method for the identification of animals vaccinated against
       brucella

<130>  EP1641.538

<150>  PCT/ES10/70784
<151>  2010-11-29

<160>  3

<170>  PatentIn version 3.5

<210>  1
<211>  225
<212>  PRT
<213>  Artificial

<220>
<223>  gfp mut 3 protein

<400>  1

Glu Glu Leu Phe Thr Gly Val Val Pro Ile Leu Val Glu Leu Asp Gly
1               5                   10                  15

Asp Val Asn Gly His Lys Phe Ser Val Ser Gly Glu Gly Glu Gly Asp
            20                  25                  30

Ala Thr Tyr Gly Lys Leu Thr Leu Lys Phe Ile Cys Thr Thr Gly Lys
        35                  40                  45

Leu Pro Val Pro Trp Pro Thr Leu Val Thr Thr Phe Gly Tyr Gly Val
    50                  55                  60

Gln Cys Phe Ala Arg Tyr Pro Asp His Met Lys Gln His Asp Phe Phe
65                  70                  75                  80

Lys Ser Ala Met Pro Glu Gly Tyr Val Gln Glu Arg Thr Ile Phe Phe
                85                  90                  95

Lys Asp Asp Gly Asn Tyr Lys Thr Arg Ala Glu Val Lys Phe Glu Gly
            100                 105                 110

Asp Thr Leu Val Asn Arg Ile Glu Leu Lys Gly Ile Asp Phe Lys Glu
        115                 120                 125

Asp Gly Asn Ile Leu Gly His Lys Leu Glu Tyr Asn Tyr Asn Ser His
    130                 135                 140

Asn Val Tyr Ile Met Ala Asp Lys Gln Lys Asn Gly Ile Lys Val Asn
145                 150                 155                 160

Phe Lys Ile Arg His Asn Ile Glu Asp Gly Ser Val Gln Leu Ala Asp
                165                 170                 175

His Tyr Gln Gln Asn Thr Pro Ile Gly Asp Gly Pro Val Leu Leu Pro
            180                 185                 190

Asp Asn His Tyr Leu Ser Thr Gln Ser Ala Leu Ser Lys Asp Pro Asn
        195                 200                 205

Glu Lys Arg Asp His Met Val Leu Leu Glu Phe Val Thr Ala Ala Gly
    210                 215                 220

Ile
225

<210>  2
<211>  20
<212>  DNA
<213>  Artificial

<220>
<223>  forward primer

<400>  2
tctcgcaaag cattgaacac                                                20

<210>  3
<211>  20
<212>  DNA
<213>  Artificial Sequence

<220>
<223>  reverse primer

<400>  3
gtcagtggag agggtgaagg                                                20